# EUROPÄISCHE PATENTSCHRIFT

(11) **EP 3 170 196 B1**
(45) Veröffentlichungstag und Bekanntmachung des Hinweises auf die Patenterteilung: **02.05.2018**
(21) Anmeldenummer: 15739229.1
(22) Anmeldetag: 13.07.2015
(51) Int. Cl.: H01J 37/16, H01J 37/32, H01L 21/67, A61L 2/14

(54) **VAKUUMANLAGE, INSBESONDERE PLASMAANLAGE, MIT EINEM RUNDUM GESCHLOSSENEN KAMMERSTRANGPRESSPROFIL**
VACUUM SYSTEM, IN PARTICULAR A PLASMA SYSTEM, HAVING A COMPLETELY ENCLOSED CHAMBER EXTRUDED PROFILE
INSTALLATION SOUS VIDE, NOTAMMENT INSTALLATION À PLASMA, AYANT UN PROFIL EXTRUDÉ DE CHAMBRE INTÉGRALEMENT FERMÉ

(30) Priorität: 17.07.2014 DE 102014213942
(43) Veröffentlichungstag der Anmeldung: 24.05.2017
(73) Patentinhaber: Diener, Christof-Herbert, 72202 Nagold (DE)
(72) Erfinder: Diener, Christof-Herbert, 72202 Nagold (DE)
(74) Vertreter: Kohler Schmid Möbus Patentanwälte
(86) Internationale Anmeldenummer: PCT/EP2015/065968
(87) Internationale Veröffentlichungsnummer: WO 2016/008844

(56) Entgegenhaltungen:
- EP-A1- 2 116 113
- EP-A2- 1 221 492
- WO-A1-84/02287
- US-A1- 2008 286 697
- US-A1- 2014 061 202
- US-B1- 6 405 423

## Beschreibung

Die Erfindung betrifft eine Vakuumanlage mit einer Vakuumkammer, bei der die parallel zur mittigen Vakuumkammerlängsachse verlaufende Aussenseite der Vakuumkammer einteilig aus einem Kammerstrangpressprofil ausgebildet ist.

Eine solche gattungsgemäße Vakuumanlage ist aus der US 6,405,423 B1 bekannt geworden. Die bekannte Vakuumanlage weist eine Vakuumkammer auf. Die Seitenwände der Vakuumkammer bestehen aus einem Kammerstrangpressprofil. Die Stirnseiten des Kammerstrangpressprofils sind durch an das Kammerstrangpressprofil angeschraubte Platten verschlossen. Das Kammerstrangpressprofil weist an zumindest einer senkrecht zur Stirnseite des Kammerstrangpressprofils ausgebildeten Außenseite, d.h. an einer Seitenwand, eine Öffnung zum Be- und Entladen der Vakuumkammer auf. Die Öffnung muss aufwändig in das Kammerstrangpressprofil gefräst werden.

Aufgabe der vorliegenden Erfindung ist demgegenüber, eine Vakuumanlage bereitzustellen, deren Vakuumkammer eine hohe Dichtheit aufweist und dennoch auf einfache und kostengünstige Art und Weise fertigbar ist.

Diese Aufgabe wird erfindungsgemäß durch eine Vakuumanlage gemäß Anspruch 1 gelöst. Die Unteransprüche geben bevorzugte Weiterbildungen an.

Die erfindungsgemäße Aufgabe wird somit gelöst durch eine Vakuumanlage mit einer Vakuumkammer, bei der die parallel zur mittigen Vakuumkammerlängsachse verlaufende Aussenseite der Vakuumkammer einteilig aus einem Kammerstrangpressprofil ausgebildet ist, wobei das Kammerstrangpressprofil quer zur mittigen Vakuumkammerlängsachse vollständig geschlossen ausgebildet ist und eine stirnseitige reversibel öffen- und schließbare Tür aufweist.

Erfindungsgemäß wird somit auf das aufwändige Fräsen einer Öffnung in das Kammerstrangpressprofil verzichtet. Vielmehr wird das Kammerstrangpressprofil quer zur mittigen Vakuumkammerlängsachse rundum geschlossen ausgebildet. Das Beladen und Entladen der Vakuumkammer erfolgt über dessen Stirnseite die in Form einer reversibel öffen- und schließbaren Tür ausgebildet ist. Im Gegensatz zur US 6,405,423 B1 wird das Kammerstrangpressprofil somit bei der erfindungsgemäßen Vakuumanlage nicht vertikal stehend, sondern horizontal liegend eingesetzt. Die mittige Vakuumkammerlängsachse verläuft daher im Betrieb der Vakuumanlage horizontal. Insgesamt kann die erfindungsgemäße Vakuumanlage hierdurch konstruktiv deutlich einfacher ausgebildet und somit kostengünstiger gefertigt werden.
Die Vakuumkammer kann an einer ersten Stirnseite eine auflegbare Tür aufweisen. Eine solche auflegbare Tür kann zum Verschluss der Vakuumkammer mittels einer Pumpe angesaugt werden. Alternativ dazu kann die Vakuumkammer an einer ersten Stirnseite eine Schiebetür aufweisen. Sowohl im Falle einer auflegbaren Tür als auch im Falle einer schiebbaren Tür kann die Vakuumkammer scharnierfrei ausgebildet werden. Erfidungsgemäß weist die Vakuumkammer jedoch an einer ersten Stirnseite eine schwenkbar öffen- und schließbare Tür auf. Die Schwenkachse der Tür verläuft dabei bevorzugt quer zur mittigen Vakuumkammerlängsachse, insbesondere senkrecht zu einer Achse parallel zur mittigen Vakuumkammerlängsachse. Die Vakuumanlage kann dadurch konstruktiv besonders einfach und kostengünstig ausgebildet werden.
Zur Ausbildung einer stirnseitig schwenkbaren Tür an dem Kammerstrangpressprofil weist das das Kammerstrangpressprofil erfindungsgemäß an seiner Aussenseite einen parallel zur mittigen Vakuumkammerlängsachse verlaufenden ersten Scharniervorsprung auf, an dem ein erstes Scharnier zur schwenkbaren Verbindung zwischen Tür und Kammerstrangpressprofil angeordnet ist. Durch den ersten Scharniervorsprung wird die Montage eines ersten Scharniers deutlich erleichtert.
Das Kammerstrangpressprofil kann an seiner Aussenseite einen parallel zur mittigen Vakuumkammerlängsachse verlaufenden zweiten Scharniervorsprung aufweisen, an dem ein zweites Scharnier zur schwenkbaren Verbindung zwischen Tür und Kammerstrangpressprofil angeordnet ist. Das zweite Scharnier festigt die schwenkbare Verbindung zwischen Tür und Kammerstrangpressprofil, so dass die Dichtheit zwischen Tür und Kammerstrangpressprofil signifikant erhöht wird.

Zum Schließen der Tür am Kammerstrangpressprofil kann die Vakuumanlage einen ersten Verschluss aufweisen, der an einem ersten Verschlusshaltervorsprung angeordnet ist, wobei der Verschlusshaltervorsprung parallel zur mittigen Vakuumkammerlängsachse an der Aussenseite des Kammerstrangpressprofils ausgebildet ist.

In bevorzugter Ausgestaltung der Erfindung weist das Kammerstrangpressprofil an seiner Aussenseite einen parallel zur mittigen Vakuumkammerlängsachse verlaufenden zweiten Verschlusshaltervorsprung auf, an dem ein zweiter Verschluss zum Schließen der Tür am Kammerstrangpressprofil angeordnet ist. Durch den zweiten Verschluss wird die Dichtheit zwischen Tür und Kammerstrangpressprofil weiter erhöht.

Das Kammerstrangpressprofil kann weiterhin an seiner Aussenseite eine parallel zur mittigen Vakuumkammerlängsachse verlaufende Kühlrippe aufweisen. Bevorzugt weist das Kammerstrangpressprofil an seiner Aussenseite eine Vielzahl von parallel zur mittigen Vakuumkammerlängsachse verlaufenden Kühlrippen auf. Durch die Kühlrippe(n) kann in vielen Anwendungen auf eine Wasserkühlung verzichtet werden.

Die Vakuumanlage weist bevorzugt ein Gehäuse auf. Zur Befestigung der Vakuumkammer an diesem Gehäuse kann das Kammerstrangpressprofil an seiner Aussenseite einen parallel zur mittigen Vakuumkammerlängsachse verlaufenden ersten Befestigungsvorsprung aufweisen. Der erste Befestigungsvorsprung erleichtert die Montage der Vakuumkammer am Gehäuse, so dass Montagekosten gesenkt werden.

Bevorzugt weist das Kammerstrangpressprofil an seiner Aussenseite einen parallel zur mittigen Vakuumkammerlängsachse verlaufenden zweiten Befestigungsvorsprung zur Befestigung der Vakuumkammer an dem Gehäuse der Vakuumanlage auf. Durch den zweiten Befestigungsvorsprung wird die Vakuumkammer konstruktiv besonders einfach und gleichzeitig sicher am Gehäuse der Vakuumanlage befestigt.

In weiterer bevorzugter Ausgestaltung der Erfindung weist das Kammerstrangpressprofil an seiner Innenseite zwei sich gegenüberliegende parallel zur mittigen Vakuumkammerlängsachse verlaufende Einschubausnehmungen zum Einschub einer Platte auf. Hierdurch wird die Herstellung einer Probenhalterung im Inneren einer Vakuumkammer deutlich vereinfacht.

Vorzugsweise weist das Kammerstrangpressprofil an seiner Innenseite mehrere sich gegenüberliegende parallel zur mittigen Vakuumkammerlängsachse verlaufende Einschubausnehmungen zum Einschub von Platten auf. Bereits bei der Herstellung des Kammerstrangpressprofils werden hierdurch mehrere Plattenhalterungen gefertigt, wodurch die Herstellung der Vakuumanlage deutlich vereinfacht wird.

Eine der ersten Stirnseite gegenüberliegende zweite Stirnseite des Strangpressprofils kann einen Flansch zur Einführung und oder Ausführung eines Mediums in bzw. aus der Kammer aufweisen.

Eine besonders einfache und kostengünstige Herstellung der Vakuumkammer erfolgt, wenn das Kammerstrangpressprofil aus Aluminium oder einer Aluminiumlegierung ausgebildet ist.

Die Herstellung der Vakuumanlage wird weiterhin signifikant vereinfacht, wenn die Vakuumanlage ein Gehäuse aufweist, in dem die Vakuumkammer aufgenommen ist, wobei ein parallel zur mittigen Vakuumkammerlängsachse verlaufender Teil des Gehäuses in Form eines Gehäusestrangpressprofils ausgebildet ist. Mit anderen Worten wird die Herstellung der Vakuumanlage dadurch vereinfacht, dass nicht nur ein maßgeblicher Teil der Vakuumkammer, nämlich das Kammerstrangpressprofil, sondern auch ein Teil des Gehäuses, nämlich das Gehäusestrangpressprofil, durch ein Strangpressverfahren herstellbar ist.

In weiterer bevorzugter Ausgestaltung der Erfindung ist das Kammerstrangpressprofil einteilig mit dem Gehäuse des Strangpressprofil ausgebildet.

Die Vakuumanlage kann in Form eines Vakuumtrockners ausgebildet seit.

Die Vakuumanlage kann hierzu eine Pumpe zur zumindest teilweisen Evakuierung der Vakuumkammer aufweisen. Weiterhin kann die Vakuumanlage eine Heizung aufweisen.

Das Strangpressprofil kann zumindest an seiner Innenseite vernickelt sein, um Korrosion zu vermeiden. Bevorzugt ist die gesamte Vakuumkammer an ihrer Innenseite vernickelt.

In weiterer bevorzugter Ausgestaltung der Erfindung ist die Vakuumanlage in Form einer Plasmaanlage ausgebildet. Im Inneren der Vakuumkammer kann in diesem Fall zumindest eine Plasmaelektrode angeordnet sein. Die Vakuumanlage in Form einer Plasmaanlage kann weiterhin eine Pumpe, ein Belüftungsventil, einen Gasflussmesser, eine Druckanzeige und/oder eine Hochspannungsversorgung aufweisen.
Zusammenfassend sind folgende Varianten zum Einsatz eines Strangpressprofils im Rahmen der erfindungsgemäßen Vakuumanlage denkbar:
a) Die Vakuumkammer weist ein Kammerstrangpressprofil auf;
b) Die Vakuumanlage weist ein Gehäusestrangpressprofil auf.
Das Kammerstrangpressprofil kann mit dem Gehäusestrangpressprofil verbunden sein. Beispielsweise kann das Kammerstrangpressprofil mit dem Gehäusestrangpressprofil verschweißt und/oder verschraubt sein. Alternativ dazu können das Kammerstrangpressprofil und das Gehäusestrangpressprofil einteilig, d.h. einstückig, ausgebildet sein. Weitere Merkmale und Vorteile der Erfindung ergeben sich aus der nachfolgenden detaillierten Beschreibung mehrerer Ausführungsbeispiele der Erfindung, anhand der Figuren der Zeichnung, die erfindungswesentliche Einzelheiten zeigt sowie aus den Ansprüchen. Die in der Zeichnung gezeigten Merkmale sind derart dargestellt, dass die erfindungsgemäßen Besonderheiten deutlich sichtbar gemacht werden können.

Es zeigen:
- Figur 1: eine perspektivische Ansicht einer Vakuumanlage in Form einer Plasmaanlage;
- Figur 2: eine Draufsicht auf ein erstes Kammerstrangpressprofil;
- Figur 3: eine Draufsicht auf das erste Kammerstrangpressprofil gemäß Figur 2, wobei das erste Kammerstrangpressprofil an einem ersten Gehäusestrangpressprofil befestigt ist; und
- Figur 4: eine Draufsicht auf ein zweites Kammerstrangpressprofil das einteilig mit einem zweiten Gehäusestrangpressprofil ausgebildet ist.

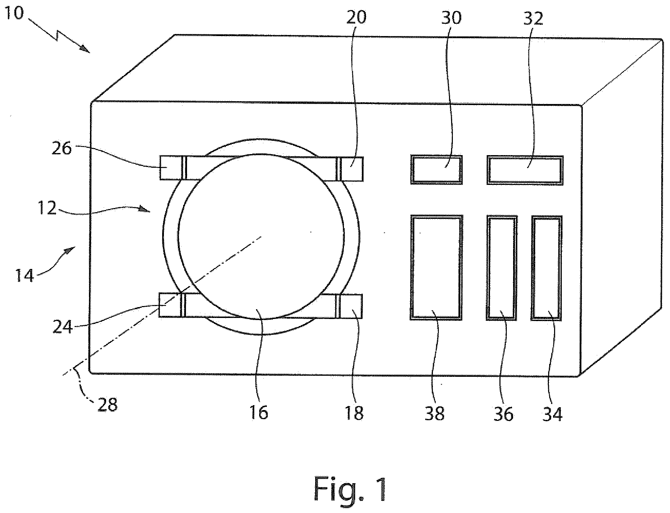

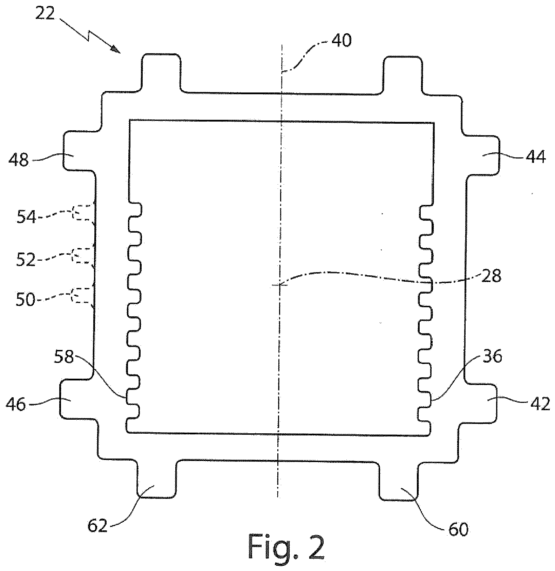

Figur 1 zeigt eine Vakuumanlage 10 mit einer Vakuumkammer 12. Die Vakuumkammer 12 ist weitestgehend von einem Gehäuse 14 umgeben. Die Vakuumkammer 12 weist eine Tür 16 auf die durch Scharniere 18, 20 schwenkbar öffen- und schließbar ist. Die Scharniere 18, 20 sind einen Ends an der Tür 16 und anderen Ends an einem Kammerstrangpressprofil 22 (siehe Figur 2) befestigt. Zum Schließen der Tür 16 an dem Kammerstrangpressprofil 22 sind Verschlüsse 24, 26 vorgesehen. Die Verschlüsse 24, 26 sind einen Ends am Kammerstrangpressprofil 22 (siehe Figur 2) befestigt und greifen anderen Ends bei verschlossener Tür 16 in entsprechende Halterungen der Tür 16 ein.

Die Vakuumkammer 12 ist horizontal angeordnet, d.h. eine mittige Vakuumkammerlängsachse 28 verläuft im Betriebszustand der Vakuumanlage 10 in horizontaler Richtung.

Die Vakuumanlage 10 weist eine Druckanzeige 30, einen Timer 32, Gasflusssteuereinrichtungen 34, 36 sowie eine Hochspannungssteuerung 38 auf. Die Druckanzeige 30 dient der Anzeige des Innendrucks der Vakuumkammer 12. Der Timer 32 dient der Einstellung einer Prozesszeit. Durch die Gasflussmesseinrichtungen 34, 36 kann der Gasfluss in die Vakuumkammer 12 eingestellt und kontrolliert werden. Die Hochspannungssteuerung 38 dient der Spannungsversorgung einer Plasmaelektrode (nicht gezeigt) in der Vakuumkammer 12.

Figur 2 zeigt das Kammerstrangpressprofil 22. Das Kammerstrangpressprofil 22 ist gleichförmig in Richtung der mittigen Vakuumkammerlängsachse 28 ausgebildet, die in Figur 2 lediglich punktförmig gezeigt ist. Weiterhin ist das Kammerstrangpressprofil 22 symmetrisch zu einer mittigen Vakuumkammerlängsebene 40 ausgebildet. Das Kammerstrangpressprofil 22 ist aus Aluminium ausgebildet.

Das Kammerstrangpressprofil 22 weist an seiner Aussenseite einen ersten Scharniervorsprung 42 und einen zweiten Scharniervorsprung 44 auf. Die Scharniere 18, 20 (siehe Figur 1) sind an den Scharniervorsprüngen 42, 44 montiert. Das Kammerstrangpressprofil 22 weist weiterhin einen ersten Verschlusshaltervorsprung 46 auf, an dem der erste Verschluss 24 (siehe Figur 1) montiert ist. Der zweite Verschluss 26 (siehe Figur 1) ist an einem zweiten Verschlusshaltervorsprung 48 befestigt. Die Vorsprünge 42, 44, 46, 48 ermöglichen eine konstruktiv einfache und gleichzeitig sehr dichte Anordnung der Tür 16 (siehe Figur 1) an dem Kammerstrangpressprofil 22 in schwenkbarer Anordnung.

Zur Kühlung des Kammerstrangpressprofils 22 kann dieses an seiner Aussenseite eine oder mehrere Kühlrippen 50, 52, 54 (in Figur 2 gestrichelt dargestellt) aufweisen.

Das Kammerstrangpressprofil 22 weist an seiner Innenseite mehrere Einschubausnehmungen auf, in die jeweils eine Platte (nicht gezeigt) eingeführt werden kann. Aus Gründen der Übersichtlichkeit sind in Figur 2 lediglich eine erste Einschubausnehmung 56 und eine zweite Einschubausnehmung 58 mit einem Bezugszeichen versehen. Die Einschubausnehmungen 56, 58 sind einander gegenüberliegend symmetrisch zur Vakuumkammerlängsebene 40 ausgebildet.

Zur Befestigung des Kammerstrangpressprofils 22 an dem Gehäuse 14 (siehe Figur 1) weist das Kammerstrangpressprofil 22 Befestigungsvorsprünge 60, 62 auf.

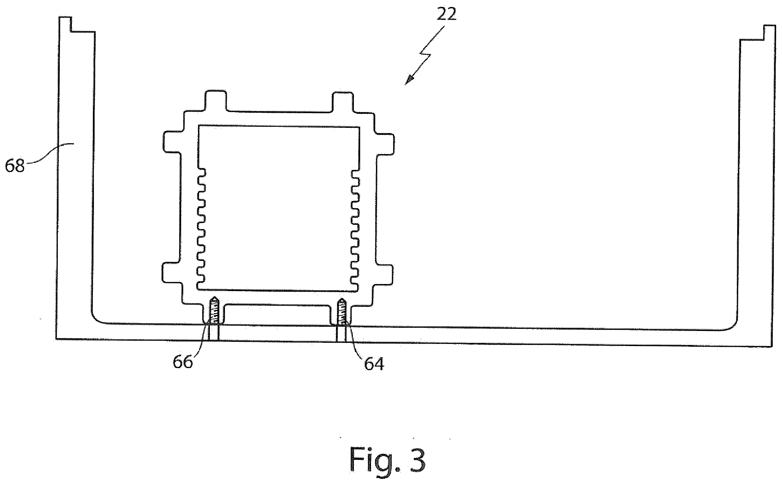

Figur 3 zeigt das Kammerstrangpressprofil 22 das über Befestigungsmittel 64, 66 an einem Gehäusestrangpressprofil 68 montiert ist. Das Gehäuse-strangpressprofil 68 stellt einen Teil des Gehäuses 14 (siehe Figur 1) dar. Im vorliegenden Fall sind die Befestigungsmittel 64, 66 in Form von Schrauben ausgebildet.

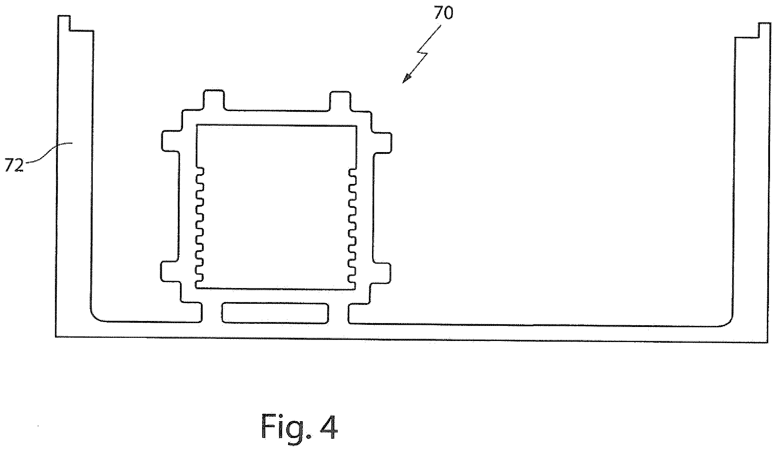

Figur 4 zeigt ein zweites Kammerstrangpressprofil 70 sowie ein zweites Gehäusestrangpressprofil 72, wobei die Strangpressprofile 70, 72 im Gegensatz zu den Strangpressprofilen 22, 68 (siehe Figur 3) einteilig miteinander ausgebildet sind. Hierdurch kann eine besonders effiziente Fertigung einer Vakuumanlage erreicht werden.

Zusammenfassend betrifft die Erfindung eine Vakuumanlage, insbesondere in Form einer Plasmaanlage. Die Vakuumanlage weist eine Vakuumkammer auf. Die Seitenwände der Vakuumkammer sind in Form eines durchgängig geschlossenen Kammerstrangpressprofils ausgebildet. Eine erste Stirnseite des Kammerstrangpressprofils ist bevorzugt mit einer Platte verschlossen. Eine zweite Stirnseite des Kammerstrangpressprofils, die der ersten Stirnseite gegenüberliegt, weist eine reversibel öffen- und schließbare Tür auf. Die Tür ist durch zumindest ein Scharnier schwenkbar an dem Kammerstrangpressprofil befestigt. Hierzu weist das Kammerstrangpressprofil an seiner Außenseite einen parallel zur mittigen Vakuumkammerlängsachse verlaufenden Scharniervorsprung auf, an dem ein Scharnier angeordnet ist. Die quer zur Längsachse des Kammerstrangpressprofils vollständig geschlossenen Seitenwände ermöglichen eine einfache und kostengünstige Fertigung der Vakuumkammer. Bevorzugt ist die Vakuumkammer zumindest teilweise in einem Gehäuse aufgenommen, das zumindest teilweise ebenfalls in Form eines Strangpressprofils ausgebildet ist.

## Patentansprüche

1. Vakuumanlage (10) mit einer Vakuumkammer (12), bei der die parallel zur mittigen Vakuumkammerlängsachse (28) verlaufende Außenseite der Vakuumkammer (12) einteilig aus einem Kammerstrangpressprofil (22, 70) ausgebildet ist, **dadurch gekennzeichnet, dass** das Kammerstrangpressprofil (22, 70) quer zur mittigen Vakuumkammerlängsachse (28) vollständig geschlossenen ausgebildet ist und eine stirnseitige reversibel öffen- und schließbare Tür aufweist, wobei die Vakuumkammer (12) an einer Stirnseite eine schwenkbar öffen- und schließbare Tür (16) aufweist, wobei das Kammerstrangpressprofil (22, 70) an seiner Außenseite einen parallel zur mittigen Vakuumkammerlängsachse (28) verlaufenden Scharniervorsprung (42, 44) aufweist, an dem ein Scharnier (18, 20) zur schwenkbaren Verbindung zwischen Tür (16) und Kammerstrangpressprofil (22, 70) angeordnet ist.

2. Vakuumanlage nach Anspruch 1, bei der das Kammerstrangpressprofil (22, 70) an seiner Außenseite einen parallel zur mittigen Vakuumkammerlängsachse (28) verlaufenden Verschlusshaltervorsprung (46, 48) aufweist, an dem ein Verschluss (24, 26) zum Schließen der Tür (16) am Kammerstrangpressprofil (22, 70) angeordnet ist.

3. Vakuumanlage nach einem der vorhergehenden Ansprüche, bei der das Kammerstrangpressprofil (22, 70) an seiner Außenseite eine parallel zur mittigen Vakuumkammerlängsachse (28) verlaufende Kühlrippe (50, 52, 54) aufweist.

4. Vakuumanlage nach einem der vorhergehenden Ansprüche, bei der das Kammerstrangpressprofil (22, 70) an seiner Außenseite einen parallel zur mittigen Vakuumkammerlängsachse (28) verlaufenden Befestigungsvorsprung (60, 62) zur Befestigung der Vakuumkammer (12) an einem Gehäuse (14) der Vakuumanlage (10) aufweist.

5. Vakuumanlage nach einem der vorhergehenden Ansprüche, bei der das Kammerstrangpressprofil (22, 70) an seiner Innenseite zwei sich gegenüberliegende, parallel zur mittigen Vakuumkammerlängsachse ((28) verlaufende Einschubausnehmungen (56, 58) zum Einschub einer Platte aufweist.

6. Vakuumanlage nach einem der vorhergehenden Ansprüche, bei der die Vakuumanlage (10) ein Gehäuse (14) aufweist, in dem die Vakuumkammer (12) aufgenommen ist, wobei ein parallel zur mittigen Vakuumkammerlängsachse (28) verlaufender Teil des Gehäuses (14) in Form eines Gehäusestrangpressprofils (68, 72) ausgebildet ist.

7. Vakuumanlage nach einem der Ansprüche 1 bis 6, bei der die Vakuumanlage in Form eines Vakuumtrockners ausgebildet ist.

8. Vakuumanlage nach einem der Ansprüche 1 bis 6, bei der die Vakuumanlage (10) in Form einer Plasmaanlage ausgebildet ist.

## Claims

1. A vacuum system (10) having a vacuum chamber (12), in which that outer side of the vacuum chamber (12) which runs parallel to the vacuum chamber central longitudinal axis (28) is formed in one piece from a chamber extruded profile (22, 70), **characterized in that** the chamber extruded profile (22, 70) is configured such that it is fully closed transversely to the vacuum chamber central longitudinal axis (28) and has an end-face door which can be reversibly opened and closed, wherein the vacuum chamber (12) has on an end face a pivotably openable and closable door (16), wherein the chamber extruded profile (22, 70) has on its outer side a hinge projection (42, 44), which runs parallel to the vacuum chamber central longitudinal axis (28) and on which is disposed a hinge (18, 20) for the pivotable connection between door (16) and chamber extruded profile (22, 70).

2. The vacuum system as claimed in claim 1, in which the chamber extruded profile (22, 70) has on its outer side a latch holder projection (46, 48), which runs parallel to the vacuum chamber central longitudinal axis (28) and on which is disposed a latch (24, 26) for the closing of the door (16) on the chamber extruded profile (22, 70).

3. The vacuum system as claimed in one of the preceding claims, in which the chamber extruded profile (22, 70) has on its outer side a cooling rib (50, 52, 54) running parallel to the vacuum chamber central longitudinal axis (28).

4. The vacuum system as claimed in one of the preceding claims, in which the chamber extruded profile (22, 70) has on its outer side a fastening projection (60, 62), running parallel to the vacuum chamber central longitudinal axis (28), for fastening the vacuum chamber (12) to a housing (14) of the vacuum system (10).

5. The vacuum system as claimed in one of the preceding claims, in which the chamber extruded profile (22, 70) has on its inner side two mutually opposing insertion recesses (56, 58), running parallel to the vacuum chamber central longitudinal axis (28), for the insertion of a plate.

6. The vacuum system as claimed in one of the preceding claims, in which the vacuum system (10) has a housing (14) in which the vacuum chamber (12) is accommodated, wherein a part of the housing (14) which runs parallel to the vacuum chamber central longitudinal axis (28) is configured in the form of housing extruded profile (68, 72).

7. The vacuum system as claimed in one of claims 1 to 6, in which the vacuum system is configured in the form of a vacuum dryer.

8. The vacuum system as claimed in one of claims 1 to 6, in which the vacuum system (10) is configured in the form of a plasma system.

## Revendications

1. Installation (10) à dépression, équipée d'une chambre à dépression (12) et dans laquelle la face extérieure de ladite chambre à dépression (12), s'étendant parallèlement à l'axe médian longitudinal (28) de ladite chambre à dépression, est réalisée d'un seul tenant en un profilé extrudé de cloisonnement (22, 70), **caractérisée par le fait que** le profilé extrudé de cloisonnement (22, 70) est de réalisation intégralement fermée transversalement par rapport à l'axe médian longitudinal (28) de la chambre à dépression, et est pourvu d'une porte frontale pouvant être ouverte et fermée de manière réversible, ladite chambre à dépression (12) étant dotée, au niveau d'une face extrême, d'une porte (16) pouvant être ouverte et fermée par pivotement, ledit profilé extrudé de cloisonnement (22, 70) étant muni, à sa face extérieure, d'une saillie (42, 44) de rattachement articulé qui s'étend parallèlement à l'axe médian longitudinal (28) de ladite chambre à dépression, et sur laquelle est disposée une charnière (18, 20) dévolue à la liaison pivotante entre ladite porte (16) et ledit profilé extrudé de cloisonnement (22, 70).

2. Installation selon la revendication 1, dans laquelle le profilé extrudé de cloisonnement (22, 70) est muni, à sa face extérieure, d'une saillie (46, 48) de retenue d'une occultation, qui s'étend parallèlement à l'axe médian longitudinal (28) de la chambre à dépression et sur laquelle est disposée une occultation (24, 26) dévolue à la fermeture de la porte (16) sur ledit profilé extrudé de cloisonnement (22, 70).

3. Installation selon l'une des revendications précédentes, dans laquelle le profilé extrudé de cloisonnement (22, 70) est muni, à sa face extérieure, d'une ailette de refroidissement (50, 52, 54) qui s'étend parallèlement à l'axe médian longitudinal (28) de la chambre à dépression.

4. Installation selon l'une des revendications précédentes, dans laquelle le profilé extrudé de cloisonnement (22, 70) est muni, à sa face extérieure, d'une saillie de fixation (60, 62) qui s'étend parallèlement à l'axe médian longitudinal (28) de la chambre à dépression, en vue de la fixation de ladite chambre à dépression (12) à un carter (14) de ladite installation (10) à dépression.

5. Installation selon l'une des revendications précédentes, dans laquelle le profilé extrudé de cloisonnement (22, 70) est muni, à sa face intérieure, de deux évidements d'insertion (56, 58) placés en vis-à-vis et s'étendant parallèlement à l'axe médian longitudinal (28) de la chambre à dépression, en vue de l'insertion d'un plateau.

6. Installation selon l'une des revendications précédentes, ladite installation (10) à dépression étant équipée d'un carter (14) dans lequel la chambre à dépression (12) est logée, sachant qu'une partie dudit carter (14), s'étendant parallèlement à l'axe médian longitudinal (28) de ladite chambre à dépression, est réalisée sous la forme d'un profilé extrudé d'enveloppement (68, 72).

7. Installation selon l'une des revendications 1 à 6, ladite installation à dépression étant réalisée sous la forme d'un séchoir à dépression.

8. Installation selon l'une des revendications 1 à 6, ladite installation (10) à dépression étant réalisée sous la forme d'une installation à plasma.
